# EUROPEAN PATENT SPECIFICATION

(11) **EP 0 743 043 B1**
(45) Date of publication and mention of the grant of the patent: **10.09.2003**
(21) Application number: 96201221.7
(22) Date of filing: 02.05.1996
(51) Int. Cl.: A61B 5/11, A01K 11/00

(54) **An animal activity meter**
Messeinrichtung für die Aktivität von Tieren
Dispositif de mesure du taux d'activité d'animaux

(30) Priority: 17.05.1995 NL 1000380
(43) Date of publication of application: 20.11.1996
(60) Divisional application: 03076259.5
(73) Proprietor: MAASLAND N.V., 3155 PD Maasland (NL)
(72) Inventor: Van der Lely, Olaf, 6312 Steinhausen (CH); Van der Lely, Alexander, 6340 Baar (CH); Van den Berg, Karel, 2971 BR Bleskensgraaf (NL)
(74) Representative: Corten, Maurice Jean F.M.

(56) References cited:
- EP-A- 0 638 231
- WO-A-94/19931
- GB-A- 2 136 619
- US-A- 4 247 758
- US-A- 4 618 861

## Description

The present invention relates to a system for measuring the activity of an animal an animal activity meter according to the preamble of claim 1.

Such a system is known from US-A-4,618,861. In this system every time an animal comes in the immediate vicinity of the sensor, there is read out a count representing the detected movements of the animal. When the animals are supposed to be milked twice per twenty-four hours and, consequently, to go twice a day to the milking parlour, where the sensor for reading out the counts will be arranged in practice, there are obtained two counts per twenty-four hours. These counts are averaged over a number of days; with the average value thus obtained every new count can be compared. When a new count is sufficiently above this average value, this will be an indication of the heat of the animal. When a new count is sufficiently below this average value, this will be an indication that the animal has an injured leg or is ill, or at least has contracted a disease. In this manner, by means of such an animal activity meter, it will be possible to monitor the state of health in general and diseases, injuries and the state of heat in particular.

The invention aims at providing an alternative system for measuring the activity of an animal. Hereto the above-described system comprises according to the invention the characterizing features of claim 1. In other words, the animal's movements can continually be counted over a pre-fixed period of time of e.g. five minutes, a quarter of an hour, half an hour, while, when this period of time has elapsed, the count is recorded in the memory means, so that, when the animal comes in the vicinity of the sensor, a series of counts can be read out. In this manner, the activity pattern can accurately be updated.

When an animal is lying and ruminating, the count will be very low. During this period of rest, it will not be necessary to measure the animal activity frequently. In such a situation, according to an other inventive feature, it is possible to provide the animal activity meter with a sensor for measuring the distance to the ground and for inciting a signal indicating that the animal is lying, standing or walking. In the memory means the moments can then be recorded when the animal lies down and rises.

The transponder can be active at a relatively large distance. Under such circumstances, it is possible to use a system for measuring the activity of an animal as defined in claim 4. It is thus possible to transmit the signals immediately or at least almost immediately to the computer. Consequently, there is obtained a continuous processing of the signals representing the movements of an animal; the movements are further counted in the computer, instead of in the counting device provided in the animal activity meter.

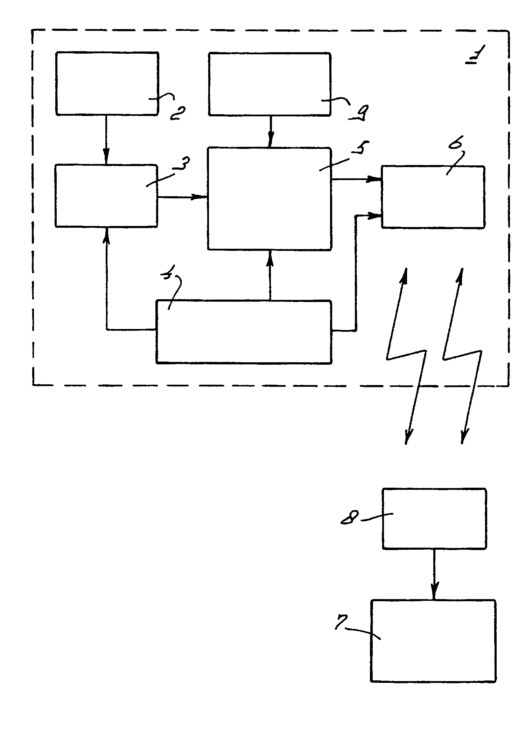

For a better understanding of the invention and to show how the same may be carried into effect, reference will now be made, by way of example, to the accompanying drawing, in which the animal activity meter according to the invention is shown schematically.

The animal activity meter 1 shown in the drawing, which meter can be worn by an animal around the neck or attached to one of the legs, is provided with a movement sensor 2, for the purpose of detecting the movements made by the animal. Said movement sensor 2 may be designed as a mercury switch. The number of movements established by means of the movement sensor 2 can be counted in a counting device 3, which is controlled by a control circuit 4 provided with a microprocessor. This control circuit ensures that the counting device 3 is reset in its starting position and will count thereafter, during a pre-fixed period of time, the number of movements made in said period of time which, optionally, can be adjusted, e.g. at five minutes, a quarter of an hour, half an hour, or at whatever value. After the adjusted period of time has elapsed, the count of the counting device is recorded in memory means 5, which are also controlled by the control circuit 4. Every time a count from the counting device 3 has been recorded in the memory means 5, the counting device 3 is reset in its starting position by the control circuit 4. In this manner, after some time, there is stored a series of counts in the memory means 5. Moreover, the animal activity meter 1 includes a transponder 6, by means of which the counts stored in the memory means 5 can be recorded in a computer 7, via a sensor 8 connected thereto. Although the sensor 8 may be arranged at a place chosen at random in a stable or cowshed, said sensor should preferably be disposed at the place where the animals are milked or near thereto, as this is the place where the animals go or are guided a number of times per twenty-four hours. As soon as the animals come in the vicinity of the sensor 8, the latter comes in contact with the transponder 6 in the animal activity meter 1, while the information present in the memory means 5 can be transmitted and recorded in the computer 7 via the sensor 8. For the purpose of supplying concentrate or for automatically milking, it is desirable that the animals can be identified at the place where they are fed or milked or near thereto. This identification is effected by means of an animal identification system provided with a transponder 6 including an electronic circuit, to be worn by the animal, in which system the animal number has been recorded, and a fixed sensor being in communication with a computer. It will be obvious that the animal activity meter and the animal identification system are adapted to be mutually integrated. For that purpose, in the embodiment shown, the memory means 5, besides the counts, also contain the animal number and, as soon as the animal comes in the vicinity of the sensor 8, these two data, via the transponder 6 and said sensor 8, are recorded in the computer 7. For each animal, there may be in the memory of the computer 7 a file, that is accessible with the aid of said animal number and in which the counts are stored. This file may additionally include all kinds of information relevant for feeding the animals or for automatically milking same.

When the animals are automatically milked while making use of a milking robot, the animals allowed to walk freely in an accommodation, such as a stable or cowshed or a meadow, can go individually, without human intervention, to the milking parlour in which the milking robot is disposed, where, after having been identified by means of the animal identification system, they may be milked. As there is usually supplied concentrate to the animals in the milking parlour, and the animals can go there voluntarily, it will be obvious that not every time the animals present themselves in the milking parlour they will actually be milked. It is possible to let the decision whether or not to milk an animal depend on the activity pattern as is established by means of the animal activity meter 1, as, for each animal, the activity pattern between two consecutive moments when the animal presents itself in the milking parlour or near thereto is established and recorded in the computer 7. When, every time the animal has presented itself in the milking parlour or near thereto, a series of counts is read out, there is obtained a permanently updated activity pattern. After having been milked, an animal will be in movement and consume fodder. After some time, the animal will lie down and ruminate. When, thereafter, the animal rises and begins to move, its movements will be less frequent than in the period prior to ruminating, as the lactation has sufficiently been resumed and the udder has become heavy, so that the freedom of movement of the animal is hampered. When the animal then goes again to the milking parlour, it will be milked. However, when the animal goes to the milking parlour and it appears from the activity pattern that this animal has not yet ruminated, then said animal has obviously presented itself too early and has to be removed from the milking parlour without having been milked. In other words, on the basis of the updated activity pattern, there can be decided whether or not to milk the animal when the latter presents itself in the milking parlour or near thereto. During the period of time the animal is lying down and is possibly ruminating, its movements are minimal. During that time, it is not strictly necessary that the counting device 3 is active and that the count is periodically recorded from said counting device in the memory means 5. The animal activity meter 1 may be provided with a specific sensor 9 for the purpose of measuring the distance to the ground and inciting a signal indicating that the animal is lying, standing or walking. The signals incited by this distance measuring sensor determine the moments when the animal lies down and rises, and can be recorded in the memory means 5. In other words, the periods of rest of the animal, obtained by means of the distance measuring sensor 9, can be stored directly in the memory means 5. These data can be recorded, in the same manner as the counts and the animal number, from the memory means 5, via the transponder 6 and the sensor 8, in the computer 7.

Besides directly from the updated activity pattern, the decision whether or not to milk an animal presenting itself in the milking parlour or near thereto can also be taken depending on the fact whether or not the sum of the counts, recorded in the computer 7 from the moment when the animal has been milked the previous time, exceeds a fixed value. After all, in the computer 7 there can be recorded how many movements a specific animal normally makes from the moment it has been milked until it has subsequently consumed fodder and has ruminated same, so that, as soon as an animal presents itself in the milking parlour or near thereto, the sum of the counts obtained since the previous milking run can be ascertained and the decision to milk the animal can be taken when this sum exceeds the threshold value for the number of movements to be made for this animal recorded in the computer.

The activity pattern detected for the individual animals can be averaged over a number of days. For example, by constantly making an average over the last week, there is obtained a possibly slowly varying value of a developing average. The animal activity as is read out from the memory means 5 every time the animal presents itself in the milking parlour or near thereto, can be compared with this developing average. In the case of values obtained that are significantly higher, the animal will be supposed to be on heat and in the case of values obtained being significantly lower, there will be an indication that the animal has one or more injured legs and/or is ill or at least has contracted a disease. Upon comparing the animal activity with the average value recorded in the memory, it has to be taken into consideration that these average values will not only differ per animal, but also depend on the fact whether the animal stays in a stable or cowshed or in a meadow. This means that for each animal there can be updated an average value for the period of time during which it stays in the stable or cowshed, as well as for the period of time during which it stays in the meadow.

The present invention is not restricted to the embodiment shown, but also relates to all kinds of modifications in the construction, of course, falling within the scope of the following claims.

## Claims

1. A system for measuring the activity of an animal, said system being provided:
with an animal activity meter (1) in which means (2) for detecting movements made by an animal and for providing signals representing these movements, a transponder (6), and a control circuit (4) for controlling the transponder (6) are incorporated,
with a device (3) for counting movements made by an animal,
with a sensor (8) and a computer (7), said sensor (8) being connected to the computer (7), and
with memory means (5) for recording the count of the counting device;
wherein said transponder (6) and said sensor (8) are suitable for transmitting signals representing the movements made by an animal to the computer (7), said computer (7) being suitable for reading out and recording said signals representing the movements made by an animal, **characterized in that** said control circuit (4) is suitable for resetting the counting device (3) after a pre-fixed period of time, and suitable for controlling the recording of the count of the counting device (3) in the memory means (5) after the pre-fixed period of time has elapsed, and **in that** said memory means (5) is suitable for storing a series of counts.

2. A system for measuring the activity of an animal as claimed in claim 1, **characterized in that** the pre-fixed period of time is adjustable.

3. A system for measuring the activity of an animal as claimed in claim 1 or 2, **characterized in that** said device (3) for counting movements made by an animal and said memory means (5) for recording the count of the counting device are incorporated in the animal activity meter (1).

4. A system for measuring the activity of an animal as claimed in claim 1 or 2, **characterized in that** said device (3) for counting movements made by an animal and said memory means (5) for recording the count of the counting device are incorporated in the computer (7).

5. A system for measuring the activity of an animal as claimed in any one of the preceding claims, **characterized in that** said animal activity meter (1) is provided with a sensor(9) for measuring the distance to the ground and for inciting a signal indicating that the animal is lying, standing or walking.

6. A system for measuring the activity of an animal as claimed in claim 5, **characterized in that** in the memory means (5) are suitable for recording the moments when an animal lies down and rises.

## Patentansprüche

1. System zum Messen der Aktivität eines Tieres, wobei das System mit folgenden Vorrichtungen versehen ist:
mit einem Tieraktivitätsmesser (1), in den eine Vorrichtung (2) zum Ermitteln von Bewegungen eines Tieres und zum Erzeugen von Signalen, die diese Bewegungen repräsentieren, ein Transponder (6) und ein Steuerkreis (4) zum Steuern des Transponders (6) integriert sind,
mit einer Vorrichtung (3) zum Zählen von Bewegungen eines Tieres,
mit einem Sensor (8) und einem Computer (7), wobei der Sensor (8) mit dem Computer (7) verbunden ist, und
mit Speichervorrichtungen (5) zum Aufzeichnen des Zählwertes der Zählvorrichtung;
wobei der Transponder (6) und der Sensor (8) dazu ausgelegt sind, Signale, die die von einem Tier gemachten Bewegungen repräsentieren, an den Computer (7) zu geben, wobei der Computer (7) dazu ausgelegt ist, die Signale, die die von einem Tier gemachten Bewegungen repräsentieren, auszulesen und aufzuzeichnen,
**dadurch gekennzeichnet, daß** der Steuerkreis (4) dazu ausgelegt ist, die Zählvorrichtung (3) nach einem vorgegebenen Zeitraum zurückzustellen, und dazu ausgelegt ist, die Aufzeichnung des Zählwertes der Zählvorrichtung (3) in der Speichervorrichtung (5) nach Ablauf des vorgegebenen Zeitraumes zu steuern, und daß die Speichervorrichtung (5) dazu ausgelegt ist, eine Reihe von Zählwerten zu speichern.

2. System zum Messen der Aktivität eines Tieres nach Anspruch 1,
**dadurch gekennzeichnet, daß** der vorgegebene Zeitraum einstellbar ist.

3. System zum Messen der Aktivität eines Tieres nach Anspruch 1 oder 2,
**dadurch gekennzeichnet, daß** die Vorrichtung (3) zum Zählen von Bewegungen eines Tieres und die Speichervorrichtung (5) zum Aufzeichnen des Zählwertes der Zählvorrichtung in den Tieraktivitätsmesser (1) integriert sind.

4. System zum Messen der Aktivität eines Tieres nach Anspruch 1 oder 2,
**dadurch gekennzeichnet, daß** die Vorrichtung (3) zum Zählen von Bewegungen eines Tieres und die Speichervorrichtung (5) zum Aufzeichnen des Zählwertes der Zählvorrichtung in den Computer (7) integriert sind.

5. System zum Messen der Aktivität eines Tieres nach einem der vorhergehenden Ansprüche,
**dadurch gekennzeichnet, daß** der Tieraktivitätsmesser (1) mit einem Sensor (9) versehen ist, der den Abstand zum Boden mißt und ein Signal gibt, das anzeigt, ob das Tier liegt, steht oder geht.

6. System zum Messen der Aktivität eines Tieres nach Anspruch 5,
**dadurch gekennzeichnet, daß** die Speichervorrichtung (5) dazu ausgelegt ist, die Zeitpunkte aufzuzeichnen, zu denen sich ein Tier hinlegt und aufsteht.

## Revendications

1. Système de mesure de l'activité d'un animal, ledit système étant pourvu :
d'un compteur d'activité de l'animal (1), dans lequel sont incorporés des moyens (2) de détection des mouvements effectués par un animal et de fourniture de signaux représentant ces mouvements, un transpondeur (6) et u n circuit de contrôle (4) pour contrôler le transpondeur (6),
d'un dispositif (3) de comptage des mouvements effectués par un animal,
d'un capteur (8) et d'un ordinateur (7), ledit capteur (8) étant relié à l'ordinateur (7), et
de supports de mémoire (5) pour enregistrer les décomptes du dispositif de comptage,
dans lequel ledit transpondeur (6) et ledit capteur (8) conviennent pour transmettre des signaux représentant les mouvements effectués par un animal à l'ordinateur (7), ledit ordinateur (7) convenant pour la restitution e t l'enregistrement desdits signaux représentant les mouvements effectués par un animal, **caractérisé en ce que** ledit circuit d e contrôle (4) convient pour remettre à zéro le dispositif d e comptage (3) au terme d'une période de durée préétablie et pour contrôler l'enregistrement du décompte du dispositif de comptage (3) dans les supports de mémoire (5) après que la période de durée préétablie se soit écoulée, et **en ce que** lesdits supports de mémoire (5) conviennent pour le stockage d'une série de décomptes.

2. Système de mesure de l'activité d'un animal selon la revendication 1, **caractérisé en ce que** la période de durée préétablie est réglable.

3. Système de mesure de l'activité d'un animal selon la revendication 1 ou 2, **caractérisé en ce que** ledit dispositif (3) de comptage des mouvements effectués par un animal et lesdits supports de mémoire (5) pour enregistrer le décompte du dispositif de comptage sont incorporés dans le compteur d'activité de l'animal (1).

4. Système de mesure de l'activité d'un animal selon la revendication 1 ou 2, **caractérisé en ce que** ledit dispositif (3) de comptage des mouvements effectués par un animal et lesdits supports de mémoire (5) pour enregistrer les décomptes du dispositif de comptage sont incorporés dans l'ordinateur (7).

5. Système de mesure de l'activité d'un animal selon l'une quelconque des revendications précédentes, **caractérisé en ce que** ledit compteur d'activité de l'animal (1) est pourvu d'un capteur (9) de mesure de la distance au sol et d'émission d'un signal indiquant si l'animal est couché, debout ou en train de. marcher.

6. Système de mesure de l'activité d'un animal selon la revendication 5, **caractérisé en ce que** les supports de mémoire (5) conviennent pour l'enregistrement des moments où l'animal se couche et de ceux où il se relève.
